# EUROPEAN PATENT SPECIFICATION

(11) **EP 1 190 712 B1**
(45) Date of publication and mention of the grant of the patent: **01.09.2004**
(21) Application number: 01870026.0
(22) Date of filing: 14.02.2001
(51) Int. Cl.: A61K 31/137, A61K 31/485, A61K 9/50, A61K 9/54

(54) **Oral once daily tramadol beads composition**
Oral anzuwendende tramadolhaltige Teilchen zur einmal täglichen Verabreichung
Composition orale de Tramadol sous forme de granules à prendre une fois par jour

(30) Priority: 22.09.2000 EP 00870214
(43) Date of publication of application: 27.03.2002
(73) Proprietor: SMB Technology, 6900 Marche en Famenne (BE)
(72) Inventor: Vanderbist, Francis, 1170 Brussels (BE); Sereno, Antonio, 1820 Melsbroek (BE); Baudier, Philippe, 1180 Uccle (BE)
(74) Representative: Powis de Tenbossche, Roland

(56) References cited:
- EP-A- 1 020 185
- WO-A-00/44353
- WO-A-97/32573
- US-A- 5 474 786
- US-A- 5 601 842

## Description

The present invention relates to a nove! oral sustained release composition of Tramadol comprising coated beads and allowing a once daily administration of the drug.

### Background of the Invention

Tramadol is a central acting analgesic agent, which possesses opioid agonist properties and activates monoaminergic spinal inhibition of pain. It has modest affinity to opioid receptors and has weak affinity to a and K receptors. Unlike the opioids, it also inhibits the reuptake of Noradrenaline and serotonine.

Tramadol may be administered orally, rectally, intravenously, subcutaneous and intramuscularly. Oral Tramadol 120mg is equipotent to oral morphine 30mg. Tramadol is generally well tolerated with dizziness, nausea, sedation, dry mouth and sweating being the principal adverse effects. Respiratory depression is uncommon.

Tramadol is white, bitter, crystalline powder. It is freely soluble in water.

Tramadol has a rapid Cₘₐₓ (30 to 60 min.) and a relatively short elimination half-life (4 to 6 hours) when administered as an immediate release formulation. In the usual treatment, four administrations per day of 80 to 100mg of Tramadol are needed to alleviate the pain consistently. Furthermore, the night is normally not completely covered and consequently patients wake up because of the reparation of the pain.

There are several patents, which described sustained release formulations of Tramadol or other opioids.

For instance, US Patent 5,919,826 discloses a sustained release of Tramadol providing a time to peak plasma levels of about 2 to 6 hours and produced by a process comprising high speed mixing of Tramadol with a hydrophobic or hydrophilic carrier having a melting point from 35 degree to 150 degree, and breaking down the agglomerates to give controlled release particles.

US Patent 5,645,858 discloses a multilayered controlled release pharmaceutical dosage form comprising a plurality of coated particles comprising a core containing Tramadol, hydroxypropyl methylcellulose, polyethyleneglycol and propylene glycol overcoated with a controlled release barrier comprising ethylcellulose and which is overcoated at least once with a mixture of Tramadol, hydroxypropyl methylcelluse, polyethyleneglycol and propyleneglycol which is overcoated with is overcoated with an additional controlled release barrier comprising ethylcellulose.

US Patent 5,639,476 discloses a controlled release dosage form comprising a substrate containing Tramadol coated with a plasticized ammonio methacylate copolymer which may contain polymerizable permeability enhancing agents, water soluble acrylic polymers, pore formers and which requires exponsure to a temperature greather than the glass transition temperature of the plasticized ammonio methacrylate copolymer for about 24 to about 60 hours to stabilize the dissolution profile.

US Patent 5,601,842 discloses a drug formulation of Tramadol or salt in an orally administrable tablet form containing at least one pharmaceutically acceptable matrixing agent selected from the group of methoxyhydroxypropylcelluloses, hydroxypropylcelluloses having a viscosity between 3.000 and 150.000mPas (2% in water 20°C).

E? 0,624,366 discloses a controlled release, oral pharmaceutical preparation in the form of a controlled release matrix suitable for dosing every twelve hours containing Tramadol incorporated in the controlled release matrix, the matrix containing between 1 and 80% of cellulose ether.

It is an object of the present invention to provide a tramadol controlled release pharmaceutical unit dosage form suitable for once daily administration containing an amount from about 50 to 500mg of tramadol or its pharmaceutical acceptable salts. said amount being calculated as tramadol hydrochloride, the dosage form containing beads comprising :
a) a core containing tramadol or its pharmaceutical acceptable salts, said core being prepared from an aqueous Tramadol blend said core comprising tableting excipient.
b) a water soluble insulating membrane separating the core from the Tramadol controlling release membrane.
c) a controlled release microporous membrane comprising at least a copolymer of ethylacrylate and methylmethacrylate and at least a pharmacologically acceptable adjuvant selected from the group consisting of pigments. fillers, wetting agents, pore formers, lubricants and antifoaming agents.

Surprisingly, it was discovered during the present invention that a water soluble insulation membrane between the core and the rate controlling releasing microporous membrane reduces the differences of Tramadol dissolution rates between batches. This phenomena is not totally understood since a water soluble membrane is unable to control the release of Tramadol.

Advantageously, the Tramadol controlled release beads comprises a core containing from 20% to 80% of Tramadol or its salts and preferably from 30 to 65% of Tramadol or its salts.

Tramadol once daily controlled release beads comprise for example a core containing Tramadol or its salts without the use of a binder.

According to an embodiment, the Tramadol once daily controlled release beads are covered by a controlled release membrane consisting of Ethyl Acrylate Methyl Methacrylate Copolymer dispersion, which does not require the use of plasticizer.

According to a detail of the invention, the Tramadol once daily controlled release bead are covered by a controlled release membrane, which does not require long curing times to stabilize.

According to a characteristic of the invention, the Tramadol once daily controlled release beads comprise a core that is manufactured by the process of extrusion spheronization.

According to a further characteristic of the invention, the Tramadol once daily controlled release beads are produced by a cost effective manufacturing process.

### Detailed Description

The present invention relates to a novel oral Tramadol controlled release unit dosage form suitable for once daily administration containing an amount from about 50 to 500mg of tramadol or its pharmaceutical acceptable salts, said amount being calculated as tramadol hydrochloride, the dosage form containing beads comprising :
a) a core containing tramadol or its pharmaceutical acceptable salts. said core being prepared from an aqueous Tramadol blend said core comprising tableting excipient.
b) a water soluble insulating membrane separating the core from the Tramadol controlling release membrane.
c) a controlled release microporous membrane comprising at least a copolymer of ethylacrylate and methylmethacrylate and at least a pharmacologically acceptable adjuvant selected from the group consisting of pigments, fillers, wetting agents. pore formers, lubricants and antifoaming agents.

More particularly, the microporous membrane comprising an ethylacrylate methylmetacrylate polymer is derived from a water dispersion of such polymer that do not require, contrary to the disclosure of US Patent 5,639,476 a curing period for about 24 to 60 hours to stabilize the dissolution profile.

More particularly, the core containing Tramadol or its pharmaceutical acceptable salts comprising tableting excipients usually used in the pharmaceutical industry is produced by well known and cost effective manufacturing processes such as extrusion - spheronization and/or agglomeration of the Tramadol onto neutral cores ("built-up process").

More particularly, the unit dose contains Tramadol controlled release beads. The unit dose may be a hard capsule and/or a tablet.

In certain preferred embodiments the unit dose comprises a hard gelatine and/or hydroxypropylcellulose capsule containing controlled release beads.

In accordance with the present invention any pharmaceutically acceptable salt of Tramadol may be used. In the preferred embodiment the hydrochloride salt of Tramadol may be used.

In another preferred embodiment the unit dose comprises hard gelatine and/or hydroxypropylmethylcellulose capsule containing controlled release beads.

Beads which are spheroids containing Tramadol or any of its salts may be produced by any conventional known method such as layering the Tramadol in solution with water over a neutral core, or layering, over a core, the Tramadol in mixture with other pharmaceutical acceptable excipients such as a binders, polymers.

The preferred method for the manufacture of beads in the present invention is the method of extrusion-spheronization. This method comprises mixing the Tramadol or its salts with a spheronizing agent such as microcrystalline cellulose and optionally other pharmaceutical excipients with water. The blend obtained is thereafter extruded trough an extruder and the extrudate is thereafter rounded with the help of a spheronizer.

In a preferred embodiment beads are produced without any binder and comprises a mixture of Tramadol hydrochloride, microcrystalline cellulose and sucrose stearate.

The insulation water soluble membrane applied onto the Tramadol containing beads before the application of the microporous membrane is constituted of any water soluble polymer such as polyvylpyrrolidone, hydroxypropyl methylcellulose, methylcellulose, hydroxypropylcellulose or mixtures therof.

The water soluble polymer or polymers may be associated into the insulation membrane with at least an adjuvant as exemplified by the following: pigments such as iron oxides and titanium dioxide; fillers such as magnesium stearate and talc; antifoaming agents such as silicone oil.

In a preferred embodiment, the insulation membrane comprises polyvinylpyrrolidone and talc.

The weigh of the insulating membrane may be 2 to 15%, preferably 3 - 8% of the weight of said core.

Sustained release beads, are obtained by coating the insulated beads, previously manufactured, with a porous membrane from which the Tramadol is liberated slowly. The microporous membrane comprises ethylacrylate and methacrylate copolymers and mixtures of water - soluble and/or water dispersible polymers and/or copolymers and may also include pharmaceutically acceptable adjuvants such as plastifying agents, pigments, fillers, wetting agents, lubricants and anti-foam agents.

The water dispersible and insoluble film forming polymers constituting the microporous membrane, comprises ethylacrylate and methylmethacrylate copolymers, such as Eudragit NE30D of Röhm Pharma (USA).

The insoluble copolymers may be associated into the microporous membrane with at least one adjutant as exemplified by the following: polyethyleneglycols, polypropyleneglycols and polyvinylpyrrolindone and soluble derivatives of cellulose such as hydroxypropylcellulose, hydroxypropylmethylcellulose, carboxymethylcellulose, and methylcellulose; Pigments such as iron oxides and titanium oxide; fillers, such as lactose and sucrose; Wetting agents, such as surfactive agents of the Span and Tween types, namely partial esters of fatty acids (lauric, palmitic, stearic and oleic acids) and anhydrides of hexitols derived from sorbitol possibly containing polyoxyethylenic chains, preferably surfactive agents of the Tween type, namely Tween 80, as well as polyethyleneglycols; lubricants, such as magnesium stearate and talc; antifoaming agents, such as silicone oil.

In addition to the polymer or copolymer, the microporous membrane contains preferably, talc and/or magnesium stearate as a lubricant, hydroxypropylmethylcellulose and polyvinylpyrrolidone as pore former, titanium dioxide as a pigment, Tween 80 as an emulsifier, and silicone oil as antifoaming agent.

Generally, the thickness of the microporous membrane is expressed by the percentage of the dry coating applied to the uncoated beads.

The weight of the microporous membrane may be 2 to 35%, preferably, 5 to 22%, of the weight of said microganules. These beads may contain the Tramadol or its salt in an amount of 20 to 80% by weight, preferably 30 to 85% by weight. The micorporous membrane may contain 5 to 95% and, preferably, 30 to 50% of polymers, polymer mixture or copolymers.

The microporous membrane for use in the present invention may be applied by coating in equipment such as fluid bed coaters, pan coaters or any suitable coating equipment.

The following examples illustrate various aspects of the present invention.

### Examples

### Example 1: Uncoated beads

| | |
|---|---|
| Tramadol Hcl | 31.8kg |
| Microcrystalline Cellulose (Avicel pH101) | 21.0kg |
| Sucrose Stearate (Crosdeta F160) | 2.17kg |
| Purified Water | 7.24kg |

In a planetary mixer collette of 160 liter capacity introduce the Tramadol Hcl, microcrystalline cellulose and the sucrose stearate, blend at speed 2 for about 15 min. Slowly add the purified water and continue mixing for an additional 15 minutes at speed 2 after all water is added. Extrude the blend through a Fuji Paudal extruder equipped with a 1mm screen. Spheronize the extrudate during about 2 minutes and dry the beads in an oven at 50°C for about 12 hours. The dried beads are screened though sieves of 1.4 and 0.7mm. The yields of beads comprised between 0.7 and 1.4mm was 44.0kg (81%).

### Example 2: Coated Beads

| **2a. Pre Coating (insulation membrane)** | |
|---|---|
| Hydroxypropylmethylcelllose | 1.35kg |
| Talc | 5.40kg |
| Purified Water | 18.0kg |

45kg of sieved beads from Example 1 were placed in a fluidized bed coater (Aeromatic). The beads were coated with 8.25kg of the coating suspension to produce the insulation membrane.

| **2b. Controlled Release Coating** | |
|---|---|
| Eudragit NE30D | 36.7kg |
| Hydroxypropylmethylcelllose | 1.08kg |
| Talc | 1.08kg |
| Polysorbate 80 | 0.216kg |
| Simethicone | 0.540kg |
| Magnesium Stearate | 0.216kg |
| Purified Water | 18.0kg |

Immediately after the insulation coating, using the same coating parameters 47.8kg of the sustained release coating was applied onto the insulated beads. Upon completion of the coating, the coated beads were placed onto trays in a drying oven for about 16 hours at 50°C. Upon cooling the coated beads were stored in containers for further use.

### Example 3: Tramadol 200mg Capsule

451mg of beads from Example 2 were filled in gelatine capsules size # 0 to produce a unit dose containing 200mg per capsule of Tramadol Hcl.

A dissolution studies was performed on the formulation of Example 3 using dissolution apparatus USP #1 (paddles) at 100 revolutions per minute. The 850ml of dissolution media was adjusted at pH values of 1.2, 4.5 and 6.8. Tramadol was measured by U. V. spectrophotometry at a wavelength of 286nm. The amount of Tramadol dissolved expressed in percent of capsule amount in function of times (hours) are summarized in Table 1.

These results clearly demonstrate that the liberation of Tramadol from the composition of Example 3 are not influenced by the pH of the dissolution media comprised between 1.2 and 6.8.

**Table 1.**

| **Dissolution results of composition of Example 3 at different pH values.** **USP Apparatus #1, 100 rpm. 850ml of dissolution medium.** | | | |
|---|---|---|---|
| **Time [h]** | **Tramadol Dissolved in Percent of Dose [%]** | | |
| | **pH 1.2** | **pH 4.5** | **pH 6.8** |
| **2** | 6 | 6.5 | 7 |
| **4** | 19 | 20 | 22 |
| **8** | 56 | 60 | 62 |
| **12** | 79 | 81 | 83 |
| **24** | 99 | 98 | 99 |

### Stability Studies

Capsules of example 3 where packaged into thermosealed aluminum (20µm) and PVC (250µm) blisters. This packaging was stored under different stability conditions and dissolution profiles were measured after exposure.

| **Dissolution results @ 100rpm, apparatus USP #1, 850ml pH 1.2 Stability Conditions** | | | | | | | | |
|---|---|---|---|---|---|---|---|---|
| **Time under Conditions [months]** | **25°C/60%RH** | | | | **30°C/60%RH** | | | |
| | 2h | 4h | 8h | 22h | 2h | 4h | 8h | 22h |
| 0 (initial) | 7 | 23 | 62 | 95 | 7 | 23 | 62 | 95 |
| 3 | 8 | 24 | 63 | 94 | 7 | 24 | 66 | 100 |
| 6 | 7 | 23 | 64 | 96 | 9 | 25 | 65 | 101 |
| 9 | 7 | 24 | 65 | 100 | 6 | 20 | 60 | 98 |
| 12 | 8 | 25 | 65 | 96 | 5 | 20 | 57 | 92 |
| 18 | 7 | 22 | 64 | 96 | ----- | ----- | ----- | ----- |

These results demonstrate that the formulation of the present invention possesses excellent dissolution stability characteristics.

### Pharmacokinetics Studies

### Single Dose Administration

The comparative bioavailability of a marketed immediate release formulation of Tramadol (4 capsules of Topalgic® 50mg in one tablet) in non food conditions and the controlled release formulation of example 3 (1 capsule containing 200mg of Tramadol) in food and non food conditions has been assessed on 29 healthy volunteers after a single dose administration. The Table 2 summarizes the pharmacokinetic parameters for Tramadol (enantiomer T₁ and T₂) for each formulation. Tram 1 and Tram 2 are both the enantiomers of Tramadol and ODT 1 and ODT 2 are both the enantiomers of the active metabolite: O-demethyl-tramadol. It is important to quantify separately both enantiomers of Tramadol and O-demethyl-tramdol since it has been proven that the different enantiomers did not have the same pharmacological and therapeutical effect.

The Table 2 summarizes the main pharmacokinetic parameters obtained after a single dose administration of 4 capsules of Topalgic® 50mg in one intake in non food conditions and the controlled release formulation of example 3 (1 capsule containing Tramadol 200mg) in fed and tasted conditions,

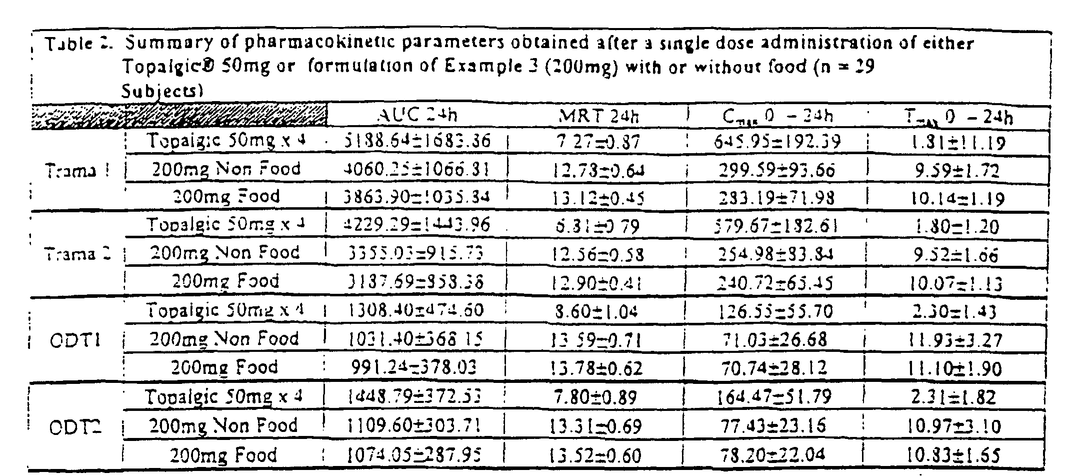

The analytical method used to quantify the amounts of Tramadol in the plasma of volunteers allowed to quantify separately both enantiomers of Tramadol (T₁ and T₂) and both enantiomers of the active metabolite of Tramadol (ODT₁ and ODT₂).

The bioanalytical method has been fully validated.

The analysis of pharmacokinetic parameters confirms that the controlled release form allows to significantly delay the Tₘₐₓ of Tramadol in comparison with immediate release form (10h versus 2h). The controlled release form also allows to significantly decrease the Cₘₐₓ of Tramadol and O-demethyl-tramadol in comparison with the immediate release (500mg/ml versus 800mg/ml). The AUC presented by the controlled release form is approximately 80% of the AUC presented by the same dose of the reference formulation.

### Repeated Dose Administration

The comparative bioavailability of a marketed immediate release formulation of Tramadol (4 capsules of Topalgic® 50mg in every 6 hours) in non food conditions and the controlled release formulation (1 capsule of Example 3 200mg) in non food conditions which is the object of the present invention has been assessed on 29 healthy volunteers after a repeated dose administration (5 days of administration).

The Table 3 summarizes the main pharmacokinetic parameters obtained after the repeated dose administration of 4 capsules of Topalgic® 50mg every 6 hours in non food conditions and the sustained release formulation (1 capsule of Example 3 200mg) in non food conditions).

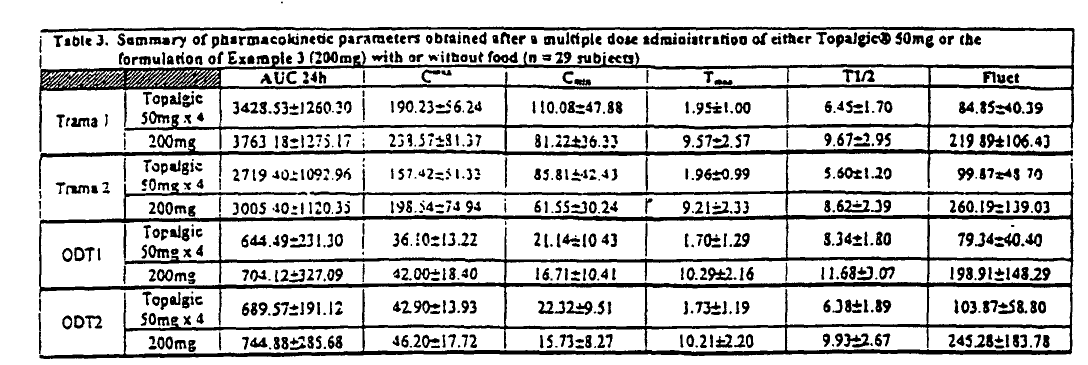

In steady-state conditions. the controlled release formulation of the present invention presents an AUC24 superior to the AUC24 of the immediate release reference formulation. The Cₘₐₓ of the formulation of the present invention after 200mg of Tramadol in one administration is only slightly superior than the Cₘₐₓ obtained after 4 administrations of 50mg of Tramadol administrated every 6 hours. This demonstrate the usefulness of the formulations of the present invention for once a day administration.

## Claims

1. A tramadol controlled release pharmaceutical unit dosage form suitable for once daily administration containing an amount from about 50 to 500mg of tramadol or its pharmaceutical acceptable salts, said amount being calculated as tramadol hydrochloride, the dosage form containing beads comprising :
a) a core containing tramadol or its pharmaceutical acceptable salts, said core being prepared from an aqueous Tramadol blend said core comprising tableting excipient.
b) a water soluble insulating membrane separating the core from the Tramadol controlling release membrane.
c) a controlled release microporous membrane comprising at least a copolymer of ethylacrylate and methylmethacrylate and at least a pharmacologically acceptable adjuvant selected from the group consisting of pigments, fillers, wetting agents, pore formers, lubricants and antifoaming agents.

2. A Tramadol controlled release pharmaceutical unit dosage form according to Claim 1 wherein the beads containing the Tramadol have a diameter, which is comprised between 0.1mm and 2mm.

3. A Tramadol controlled release pharmaceutical unit dosage form according to Claim 1 wherein the water soluble insulating membrane weight is comprised between 2% to 15% of the weight of the core.

4. A Tramadoi controlled release pharmaceutical unit dosage form according to Claim 1 wherein the microporous membrane weight, controlling the Tramadol release from the beads, is comprised between about 2% and about 35% of the weight of the core.

5. A Tramadol controlled release pharmaceutical unit dosage form according to Claim 1 wherein the beads are contained in a capsule, a bag or a dosage dispenser.

6. A Tramadol controlled release pharmaceutical unit dosage form according to Claim 1 wherein the pharmaceutical acceptable adjuvant of the microporous membrane is pigment colored selected from the group consisting of iron oxides and titanium dioxide or a filler selected from the group consisting of lactose and sucrose; or a wetting agent selected from the group consisting of fatty acid partial esters anhydrides of hexitols deviated from sorbitol contingently containing polyoxyethylenic chains and the polyethylene glycols; or a lubricant selected from the group consisting of magnesium stearate and talc; or a silicone oil as antifoaming agent or; a colloidal aluminum oxide as antistatic agent.

7. A Tramadol controlled release pharmaceutical unit dosage form according to Claim 1 wherein the water soluble insulating membrane polymer is selected from the group consisting of Providone, water soluble cellulose derivatives such as hydroxypropylcellulose, methylcellulose, hydroxyethylcellulose hydroxypropyl methylcellulose, hydroxyethyl methylcellulose, sodium carboxymethylcellulose, carboxymethylcellulose hydroxyethylcellulose and mixtrures thereof.

8. A Tramadol controlled release pharmaceutical unit dosage form according to Claim 1 wherein the water soluble insulating membrane contains adjuvants selected from the group consisting of titanium dioxide, iron oxides; or a filler selected from the group consisting of lactose, sucrose and manitol; or a lubricant selected from the group. consisting of talc and magnesium stearate; or a silicone oil antifoaming agent; or a celloidal aluminium oxide as antistatic agent.

9. A Tramadol controlled release pharmaceutical unit dosage form according to Claim 1 wherein the core, containing the Tramadol comprises between 20% to 80% of Tramadol calculated as Tramadol hydrochloride between 5% and 60% of microcrystalline cellulose and between 2% to 20% of sucrose stearate.

10. A Tramadol controlled release pharmaceutical unit dosage form according to Claim 1 wherein the core is manufactured by the process of extrusing-spheronization.

11. A Tramadol controlled release pharmaceutical unit dosage form according to Claim 1 wherein the core is manufactured by the process of "build-up" which consist of agglomerating particles and/or Tramadol in solution and optionally in mixture with other pharmaceutical excipients onto small acceptable particules.

12. A core in accordance to any of the Claim 10 and 11 wherein the core does not comprise a binder.

13. A Tramadol controlled release pharmaceutical unit dosage form according to Claim 6 wherein the microporous membrane is cured upon exposure during less than 24 hours at about 50°C.

## Patentansprüche

1. Dosisform zur kontrollierten Freisetzung einer pharmazeutischen Einheit von Tramadol geeignet zur einmal täglichen Verabreichung, die eine Menge von ungefähr 50 bis 500 mg Tramadol oder von seinen pharmazeutisch verträglichen Salzen enthält, wobei die Menge als Tramadolhydrochlorid berechnet wird, und die Dosisformenthaltenden Perlen, Kügelchen oder Körner umfassen:
a) einen Kern, der Tramadol oder seine pharmazeutisch verträglichen Salze enthält, wobei der Kern aus einer wässrigen Tramadolmischung hergestellt ist und einen tablettierenden Arzneimittelträger umfasst,
b) eine wasserlösliche Isoliermembran, die den Kern von der Tramadol-kontrollierenden Freisetzungsmembran trennt,
c) eine mikroporöse Membran zur kontrollierten Freisetzung, die mindestens ein Ethylacrylat-Methylmethacrylat-Kopolymer und mindestens einen pharmazeutisch verträglichen Zusatzstoff umfasst, der ausgewählt ist aus der Gruppe bestehend aus Pigmenten, Füllstoffen, Benetzungsmitteln, Porenbildnern, Gleitmitteln und Schaumverhütern.

2. Dosisform zur kontrollierten Freisetzung einer pharmazeutischen Einheit von Tramadol nach Anspruch 1, in der die Tramadol-enthaltenden Perlen, Kügelchen oder Körner einen Durchmesser zwischen 0,1 mm und 2 mm haben.

3. Dosisform zur kontrollierten Freisetzung einer pharmazeutischen Einheit von Tramadol nach Anspruch 1, in der das Gewicht der wasserlöslichen Isoliermembran zwischen 2% und 15% des Kerngewichtes beträgt.

4. Dosisform zur kontrollierten Freisetzung einer pharmazeutischen Einheit von Tramadol nach Anspruch 1, in der das Gewicht der mikroporösen Membran, die die Tramadolfreisetzung aus den Perlen, Kügelchen oder Körner kontrolliert, zwischen ungefähr 2% und ungefähr 35% des Kerngewichtes beträgt.

5. Dosisform zur kontrollierten Freisetzung einer pharmazeutischen Einheit von Tramadol nach Anspruch 1, in der die Perlen, Kügelchen oder Körner in einer Kapsel, einem Beutel oder einem Dosierer enthalten sind.

6. Dosisform zur kontrollierten Freisetzung einer pharmazeutischen Einheit von Tramadol nach Anspruch 1, in der der pharmazeutisch verträgliche Zusatzstoff der mikroporösen Membran pigmentgefärbt ist ausgewählt aus der Gruppe bestehend aus Eisenoxiden und Titanoxiden, oder ein Füllstoff, ausgewählt aus der Gruppe bestehend aus Laktose und Sucrose, ist, oder ein Benetzungsmittel, ausgewählt aus der Gruppe bestehend aus partiellen Fettsäureesteranhydriden von von Sorbitol abgeleiteten Hexitolen mit geringen Anteilen an Polyoxyethylenketten und Polyethylenglykolen, ist, oder ein Gleitmittel ist ausgewählt aus der Gruppe bestehend aus Magnesiumstearat und Talk, oder Silikonöl als Schaumverhüter ist, oder ein kolloides Aluminiumoxid als antistatisches Agens ist.

7. Dosisform zur kontrollierten Freisetzung einer pharmazeutischen Einheit von Tramadol nach Anspruch 1, in der das wasserlösliche Isoliermembranpolymer ausgewählt ist aus der Gruppe bestehend aus Providon, wasserlöslichen Zellulosederivaten wie Hydroxypropylzellulose, Methylzellulose, Hydroxyethylzellulose Hydroxypropylmethylzellulose, Hydroxyethylmethylzellulose, Natriumcarboxymethylzellulose, Carboxymethylzellulose Hydroxyethylzellulose und Mischungen davon.

8. Dosisform zur kontrollierten Freisetzung einer pharmazeutischen Einheit von Tramadol nach Anspruch 1, in der die wasserlösliche Isoliermembran Zusatzstoffe enthält ausgewählt aus der Gruppe bestehend aus Titandioxid, Eisenoxiden, oder einen Füllstoff enthält ausgewählt aus der Gruppe bestehend aus Laktose, Sucrose und Manitol, oder ein Gleitmittel enthält ausgewählt aus der Gruppe bestehend aus Talk und Magnesiumstearat, oder ein Silikonöl als Schaumverhüter, oder kolloides Aluminiumoxid als antistatisches Agens enthält.

9. Dosisform zur kontrollierten Freisetzung einer pharmazeutischen Einheit von Tramadol nach Anspruch 1, in der der Tramadol-enthaltende Kern zwischen 20% und 80% an Tramadol enthält, das als Tramadolhydrochlorid berechnet wird, und zwischen 5% und 60% an mikrokristalliner Zellulose und zwischen 2% und 20% an Sucrosestearat enthält.

10. Dosisform zur kontrollierten Freisetzung einer pharmazeutischen Einheit von Tramadol nach Anspruch 1, in der der Kern nach dem Verfahren der Extrusion/Sphäronisation" hergestellt ist.

11. Dosisform zur kontrollierten Freisetzung einer pharmazeutischen Einheit von Tramadol nach Anspruch 1, in der der Kern nach dem Verfahren des "Aufbaus" hergestellt ist, das in einer Agglomeration von Partikeln und/oder Tramadol in Lösung und, wahlweise, in Mischung mit anderen pharmazeutischen Arzneimittelträgern auf kleine, verträgliche Partikeln besteht.

12. Kern nach Anspruch 10 oder 11, der kein Bindemittel enthält.

13. Dosisform zur kontrollierten Freisetzung einer pharmazeutischen Einheit von Tramadol nach Anspruch 6, in der die mikroporöse Membran bei einer Exposition bei ungefähr 50°C während weniger als 24 Stunden getrocknet wird.

## Revendications

1. Forme galénique unitaire pharmaceutique à libération prolongée de Tramadol appropriée pour être administrée une fois par jour contenant une quantité d'environ 50 à 500 mg de Tramadol ou de ses sels pharmaceutiquement acceptables, ladite quantité étant calculée sous la forme de chlorhydrate de Tramadol, la forme galénique contenant des granules comprenant :
a) un noyau contenant le Tramadol ou ses sels pharmaceutiquement acceptables, ledit noyau étant préparé à partir d'un mélange aqueux de Tramadol, ledit noyau comprenant un excipient pour la formation de comprimés ;
b) une membrane isolante soluble dans l'eau séparant le noyau de la membrane de libération contrôlée du Tramadol ;
c) une membrane microporeuse de libération contrôlée comprenant au moins un copolymère d'acrylate d'éthyle et de méthacrylate d'éthyle et au moins un adjuvant pharmacologiquement acceptable choisi parmi le groupe constitué de pigments, de charges, d'agents mouillants, d'agents de formation de pores, de lubrifiants et d'agents antimousse.

2. Forme galénique unitaire pharmaceutique à libération prolongée de Tramadol selon la revendication 1 dans laquelle les granules contenant le Tramadol ont un diamètre qui est compris entre 0,1 mm et 2 mm.

3. Forme galénique unitaire pharmaceutique à libération prolongée de Tramadol selon la revendication 1 dans laquelle la membrane isolante soluble dans l'eau constitue entre 2% et 15% en poids du noyau.

4. Forme galénique unitaire pharmaceutique à libération prolongée de Tramadol selon la revendication 1 dans laquelle le poids de la membrane microporeuse, contrôlant la libération du Tramadol à partir des granules, constitue entre environ 2% et environ 35% du poids du noyau.

5. Forme galénique unitaire pharmaceutique à libération prolongée de Tramadol selon la revendication 1 dans laquelle les granules sont contenus dans une capsule, un sachet ou un distributeur de dose.

6. Forme galénique unitaire pharmaceutique à libération prolongée de Tramadol selon la revendication 1 dans laquelle l'adjuvant pharmaceutique acceptable de la membrane microporeuse est un pigment coloré choisi parmi le groupe constitué des oxydes de fer et du dioxyde de titane ou une charge choisie parmi le groupe constitué du lactose et du sucrose ; ou un agent mouillant choisi parmi le groupe constitué d'anhydrides d'ester partiel d'acide gras d'hexitols dérivés du sorbitol contenant de manière subordonnée des chaînes polyoxyéthyléniques et des polyéthylèneglycols ; ou un lubrifiant choisi parmi le groupe constitué du stéarate de magnésium et du talc ; ou d'une huile de silicone comme agent antimousse ; ou un oxyde d'aluminium colloïdal comme agent antistatique.

7. Forme galénique unitaire pharmaceutique à libération prolongée de Tramadol selon la revendication 1 dans laquelle le polymère de la membrane isolante soluble dans l'eau est choisi parmi le groupe constitué de Povidone, de dérivés de cellulose solubles dans l'eau comme l'hydroxypropylcellulose, la méthylcellulose, l'hydroxyéthylcellulose, l'hydroxypropylméthylcellulose, l'hydroxyéthylméthylcellulose, la carboxyméthylcellulose de sodium, la carboxyméthylcellulose, l'hydroxyéthylcellulose et des mélanges de celles-ci.

8. Forme galénique unitaire pharmaceutique à libération prolongée de Tramadol selon la revendication 1 dans laquelle la membrane isolante soluble dans l'eau contient des adjuvants choisis parmi le groupe constitué du dioxyde de titane, des oxydes de fer ; ou une charge choisie parmi le groupe constitué du lactose, du sucrose et du mannitol ; ou un lubrifiant choisi parmi le groupe constitué du talc et du stéarate de magnésium ; ou un agent antimousse d'huile de silicone ; ou un oxyde d'aluminium colloïdal comme agent antistatique.

9. Forme galénique unitaire pharmaceutique à libération prolongée de Tramadol selon la revendication 1 dans laquelle le noyau, contenant le Tramadol, comprend entre 20% et 80% de Tramadol, calculé sous la forme de chlorhydrate de Tramadol, entre 5% et 60% de cellulose microcristalline et entre 2% et 20% de stéarate de sucrose.

10. Forme galénique unitaire pharmaceutique à libération prolongée de Tramadol selon la revendication 1 dans laquelle le noyau est fabriqué par le procédé de sphéronisation par extrusion.

11. Forme galénique unitaire pharmaceutique à libération prolongée de Tramadol selon la revendication 1 dans laquelle le noyau est fabriqué par le procédé «d'accumulation» qui consiste à agglomérer des particules et/ou le Tramadol en solution et facultativement en mélange avec d'autres excipients pharmaceutiques sur de petites particules acceptables.

12. Noyau selon l'une quelconque des revendications 10 et 11 dans lequel le noyau ne comprend pas de liant.

13. Forme galénique unitaire pharmaceutique à libération prolongée de Tramadol selon la revendication 1 dans laquelle la membrane microporeuse est durcie lors de l'exposition durant moins de 24 heures à environ 50°C.
